# FASCICULE DE BREVET EUROPEEN

(11) **EP 2 321 251 B1**
(45) Date de publication et mention de la délivrance du brevet: **29.02.2012**
(21) Numéro de dépôt: 09780717.6
(22) Date de dépôt: 16.07.2009
(51) Int. Cl.: C07C 67/08, C07C 67/54, C07C 69/68

(54) **PROCÉDÉ CONTINU D'OBTENTION D'UN ESTER LACTIQUE**
KONTINUIERLICHES VERFAHREN ZUR GEWINNUNG EINES MILCHSÄUREESTERS
CONTINUOUS PROCESS FOR OBTAINING A LACTIC ESTER

(30) Priorité: 30.07.2008 BE 200800424
(43) Date de publication de la demande: 18.05.2011
(73) Titulaire: Galactic S.A., 7760 Escanaffles (BE)
(72) Inventeur: HOTTOIS, Delphine, B-7760 Escanaffles (BE); BRUNEAU, Alexandre, B-7760 Escanaffles (BE); BOGAERT, Jean-Christophe, B-7760 Escanaffles (BE); COSZACH, Philippe, B-7760 Escanaffles (BE)
(74) Mandataire: Vandeberg, Marie-Paule L.G.
(86) Numéro de dépôt international: PCT/EP2009/059162
(87) Numéro de publication internationale: WO 2010/012606

(56) Documents cités:
- FR-A- 2 848 208
- GB-A- 234 458

## Description

### Domaine de l'invention

La présente invention concerne un procédé continu d'obtention d'un ester lactique purifié à partir d'une solution d'acide lactique en excès et d'un alcool.

### Cadre de l'invention

Les esters lactiques sont des esters hydroxylés, certains se trouvant naturellement en petites quantités dans une grande variété d'aliments incluant le vin, les agrumes, etc. Ils se présentent sous forme liquide, incolores, possédant une odeur caractéristique et sont miscibles à l'eau et dans de nombreux solvants. Ces esters lactiques sont des solvants synthétisés à partir de matières renouvelables, il s'agit par conséquent de solvants verts ou biosolvants, communément utilisés dans les préparations pharmaceutiques, comme additif alimentaire et comme arôme. Cependant ils sont employés le plus souvent en combinaison avec d'autres solvants ou en substitution de solvants toxiques.

### Etat de l'art

Les deux formes optiquement actives d'acide lactique (L-LA) et (D-LA) peuvent donner un ester lactique sous 2 formes stéréoisomériques, l'ester L-Lactique et l'ester D-Lactique.

Dans la suite du document, nous exposerons l'invention au départ d'acide lactique de forme L(+) mais elle pourra être appliquée à l'autre forme énantiomérique ou au mélange de ces dernières. Nous entendons par acide lactique toute solution aqueuse d'acide lactique ayant une pureté en acide lactique variable et de concentration pouvant varier entre 50 et 100% massique et préférablement entre 80 et 100% massique. Il est entendu que ces solutions sont des mélanges d'eau et de monomères, dimères, trimères d'acide lactique.

La présente invention est applicable à différents esters lactiques et à leurs alcools respectifs comme par exemple, le méthanol dans le cas du lactate de méthyle ou le butanol pour le lactate de butyle. Cependant, dans la suite de la description, afin d'en simplifier la compréhension, nous particulariserons l'invention à l'ester sous forme lactate d'éthyle.

La méthode de production du lactate d'éthyle la plus fréquemment utilisée industriellement se fait à partir d'acide lactique et d'éthanol, lequel pouvant également être obtenu de ressources renouvelables, via une réaction d'estérification comme décrit notamment dans les brevets FR2848208A1 et FR2848209A1. Cette estérification entre l'acide lactique et l'éthanol conduit à la formation d'eau et de lactate d'éthyle :

CH₃CHOHCOOH + C₂H₅OH ↔ CH₃CHOHCOOC₂H₅ + H₂O

La réaction d'estérification est une réaction chimique équilibrée, c'est-à-dire qu'une partie de l'eau et du lactate d'éthyle formé peuvent réagir ensemble pour redonner l'acide lactique et l'éthanol initial. Il s'agit dans ce cas d'une hydrolyse. Les réactions d'estérification ont la réputation d'être athermiques, c'est-à-dire que leur ΔᵣH°(298 K) est proche de zéro.

La réaction d'estérification est catalysée par les acides, qu'ils soient minéraux comme par exemple l'acide sulfurique, l'acide chlorhydrique, l'acide para-toluène sulfonique, ou organiques (l'acide lactique pouvant donc lui-même jouer le rôle de catalyseur).

Du fait de la polyfonctionnalité de l'acide lactique comportant une fonction carboxylique et une fonction alcool, il est possible d'observer des estérifications inter-moléculaires successives conduisant à la formation d'oligomères :

CH₃CHOHCOOH + CH₃CHOHCOOH ↔ CH₃CHOHCOOCH(CH₃)COOH + H₂O

Suivant la concentration initiale des solutions d'acide lactique monomère, il s'établit un certain équilibre de distribution entre les différents oligomères. Cette réaction d'oligomérisation est une réaction spontanée entre deux molécules d'acide lactique. Par contre, en présence d'alcool, cette réaction est moindre voire inexistante. Dans ce cadre et afin de supprimer ces réactions, les procédés industriels se déroulent en présence d'un large excès d'éthanol selon par exemple les brevets FR2848208A1 et FR2848209A1 et généralement le rapport molaire éthanol/acide lactique est supérieur à 2:1.

D'autre part, le fait que l'acide lactique s'oligomérise spontanément peut induire la formation d'oligomères de lactate d'éthyle lors de l'estérification. En effet, tout comme l'acide lactique, les différents oligomères peuvent réagir avec l'éthanol selon la réaction:

CH₃CHOHCOOCH(CH₃)COOH + C₂HsOH → CH₃CHOHCOOCH(CH₃)COOC₂H₅ + H₂O

Par ailleurs, lors de la purification du lactate d'éthyle, on peut également observer la formation d'oligomères de lactate d'éthyle obtenus par transestérification de deux molécules d'esters:

2 CH₃CHOHCOOC₂H₅ → CH₃CHOHCOOCH(CH₃)COOC₂H₅ + C₂H₅OH

Cette réaction a lieu lors du chauffage du lactate d'éthyle pendant l'étape de purification et est favorisée par une catalyse acide. Afin d'éviter cette transestérification, il sera préférable de diminuer la température de travail par exemple en travaillant sous vide.

La production du lactate d'éthyle au départ d'acide lactique et d'éthanol n'est donc pas évidente à mettre en oeuvre de part :
1. la formation spontanée d'eau suite aux différents mécanismes énoncés ci-avant durant l'estérification. Or cette dernière :
   ■ provoque sous certaines conditions l'hydrolyse du lactate d'éthyle en acide lactique,
   ■ entraîne la formation d'un azéotrope entre l'éthanol et l'eau, nécessitant une étape supplémentaire de purification de l'éthanol,
   ■ peut entraîner la formation d'un azéotrope entre le lactate d'éthyle et l'eau compliquant leur séparation,
2. la formation spontanée d'oligomères de lactate d'éthyle durant la réaction d'estérification compliquant la purification de l'ester.

### Description détaillée de l'invention

Dans la suite du texte, les pourcentages indiqués seront toujours exprimés en poids et les rapports feront référence à des rapports molaires. De même, l'acide lactique et l'éthanol sont considérés purs ou en solution aqueuse.

La présente invention pallie ces inconvénients tout en permettant d'obtenir le lactate d'éthyle purifié par estérification d'une solution d'acide lactique par l'éthanol. Le procédé est caractérisé en ce que 1°) la réaction est réalisée en présence d'un excès d'acide lactique dans lequel on introduit directement l'éthanol afin de réaliser une catalyse optimale, la réaction est dès lors auto-catalysée, 2°) on extrait en continu uniquement la phase vapeur composée d'eau, d'éthanol, de lactate d'éthyle formé ainsi que des traces d'acide lactique à une température comprise entre 110 et 140°C et préférablement entre 115 et 125°C sous un vide de 15 à 40 kPa absolu. Ce mélange gazeux est injecté à environ mi-hauteur d'une première colonne de distillation, travaillant à cette même pression réduite où il est séparé en deux fractions : une première récoltée en tête de colonne et riche en eau, éthanol et lactate d'éthyle et un second mélange obtenu en pied de colonne, riche en acide lactique. La fraction sortant en tête est ensuite dirigée environ à mi-hauteur d'une seconde colonne de distillation, travaillant également à pression réduite, préférablement inférieure à la première colonne, pour y séparer le lactate d'éthyle du mélange éthanol - eau.

D'une façon plus générale, le procédé est caractérisé en ce qu'il comporte les étapes suivantes :
a) Réaction d'estérification d'une composition comportant un alcool en présence d'une composition comportant de l'acide lactique en excès, l'excès acide étant caractérisé par un rapport molaire acide lactique/alcool compris de 1,1:1 à 50:1 ;
b) Extraction d'une phase vapeur de ce milieu réactionnel, chargée en ester lactique, alcool, eau et des traces d'acide lactique ;
c) Distillation de la phase vapeur obtenue à l'étape b) pour récupérer en tête l'ester lactique, l'alcool et l'eau ;
d) Distillation de la fraction obtenue de l'étape c) pour récupérer en pied l'ester lactique purifié.

Le procédé décrit dans la présente invention est donc caractérisé en ce que l'éthanol peut être remplacé par un alcool contenant de 1 à 12 carbones, tel que le méthanol, l'éthanol, le n-butanol, l'isobutanol, le sec-butanol, le tert-butanol, le n-propanol, l'isopropanol, le 2-éthylhexanol, le 2-éthylbutanol, l'hexanol.

L'ester lactique purifié et en particulier le lactate d'éthyle purifié obtenu par le procédé décrit dans l'invention correspond au grade conventionnel disponible sur le marché et est caractérisé par une pureté égale ou supérieure à 97%, une teneur en eau égale ou inférieure à 0,3%, une acidité inférieure à 0,1 % et une couleur inférieure à 50 Hazen.

Selon la présente invention, la réaction d'estérification peut être menée à pression atmosphérique ou sous pression, de préférence entre 15 et 300 kPa absolu et à une température comprise entre 110 et 200°C.

Le réacteur d'estérification est préalablement rempli avec une charge d'acide lactique et est chauffé à 100°C. La réaction consiste, dès 100°C, en l'ajout continu d'une solution d'acide lactique par le haut du réacteur et d'éthanol anhydre ou azéotropique, liquide ou gazeux, par le fond afin de le disperser dans tout le volume réactionnel grâce au mobile d'agitation. Tout équipement permettant d'améliorer la dispersion de l'éthanol dans le milieu (diffuseur, spray ou autre) peut être considéré dans le cadre de cette invention. De même, tout profil d'agitation permettant d'améliorer la dispersion de l'éthanol dans le milieu (agitateur mono-étagé ou multi-étagé, turbine de type « rushton » ou autre) peut être considéré dans la cadre de cette invention. Le réacteur aura idéalement une géométrie favorisant la dispersion de l'éthanol (augmentée par la hauteur de liquide) et la vaporisation de la phase gazeuse (augmentée par le diamètre du réacteur). L'emploi d'éthanol anhydre est préférable mais pas indispensable. L'éthanol est caractérisé en ce qu'il contient au minimum 30% d'éthanol. Le rapport molaire éthanol/acide lactique de ce qui est alimenté dans le réacteur d'estérification est de préférence compris entre 1 :1 et 5:1.

Dans le cadre de la présente invention, une partie de l'éthanol réagit avec l'acide lactique pour produire du lactate d'éthyle et de l'eau. Il est de plus utilisé comme agent strippant pour favoriser l'extraction des volatils du milieu réactionnel. On veillera cependant à contrôler cette fraction afin d'éviter un procédé trop énergivore de part un bouclage trop important d'éthanol.

La particularité de la présente invention réside dans le fait que :
- bien que l'alimentation des réactifs se fasse de telle manière à être en excès éthanol, de part son action de stripping, la réaction d'estérification prend place en milieu acide. L'estérification est donc rendue possible par l'extraction en continu de la phase gazeuse tout en limitant l'entraînement d'acidité,
- il n'est pas nécessaire d'employer un catalyseur, l'agent catalysant est déjà en quantité suffisante dans le milieu réactionnel vu l'excès d'acide lactique, la réaction est par conséquent auto-catalysée.

Selon ledit procédé, la fraction recueillie en tête de la première colonne présente une acidité résiduelle inférieure à 0,5% (pour 100% de lactate d'éthyle), et de préférence inférieure à 0,2% afin d'éviter l'oligomérisation de l'ester, réaction décrite précédemment. Cette valeur d'acidité peut par exemple être obtenue en ajustant le taux de reflux de la colonne de distillation.

Selon l'invention, ces condensats sortant en tête sont ensuite dirigés vers une seconde colonne de distillation où ils sont injectés environ à mi-hauteur de colonne. Ce mélange éthanol - eau - lactate d'éthyle est soumis à une distillation préférentiellement sous pression réduite, de préférence inférieure ou égale à 10 kPa absolu, de laquelle on récupère en tête de colonne un mélange éthanol - eau et en pied, le lactate d'éthyle purifié; distillation menée de sorte à réduire au maximum le temps de séjour du mélange. Le procédé de l'invention permet de produire un lactate d'éthyle purifié.

La phase éthanol - eau soutirée en tête de la seconde colonne de distillation peut être, selon un mode de réalisation, séparée dans une colonne de distillation fonctionnant sous pression réduite ou à pression atmosphérique suivant la composition de l'éthanol souhaitée. Elle peut encore être traitée par un procédé de distillation azéotropique.

Selon un autre mode de réalisation de l'invention, la déshydratation du mélange éthanol - eau peut être effectuée au moyen de la technique PSA « Pressure Switch Adsorption » et qui consiste à effectuer l'adsorption sélective de l'eau sur un lit de tamis moléculaire en y faisant passer le mélange azéotropique. L'éthanol anhydre récupéré est avantageusement recyclé dans le milieu réactionnel.

Le mélange éthanol - eau, selon un autre mode de l'invention, peut être traité par pervaporation. Il s'agit d'un procédé de séparation des constituants par vaporisation partielle au travers d'une membrane dense présentant une affinité préférentielle pour l'un des constituants.

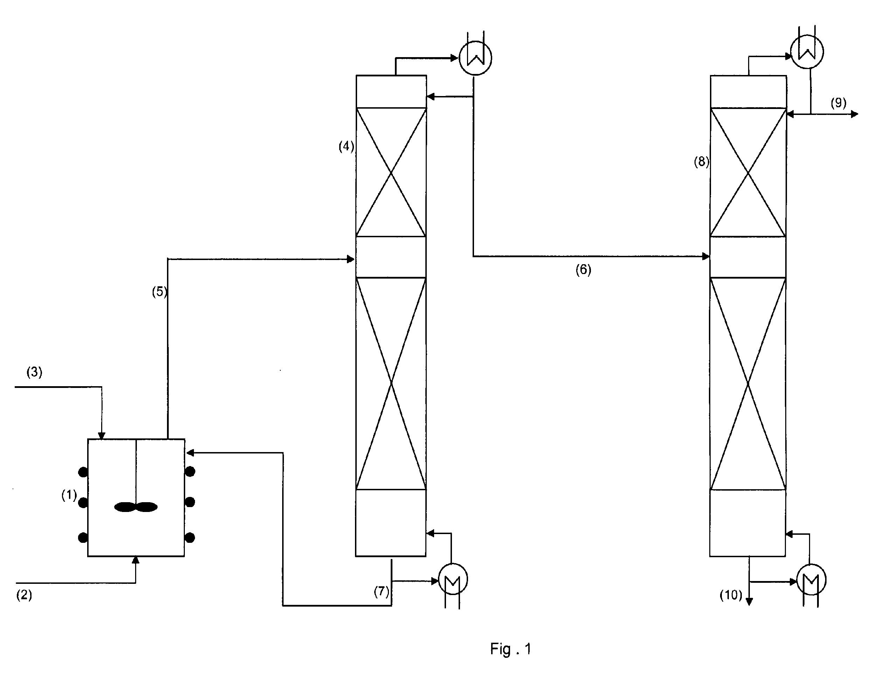

La figure 1 décrit de manière schématique un mode d'exécution particulier de l'invention. Ce dispositif comprend :
- un réacteur (1) muni d'un mobile d'agitation et éventuellement de contre-pales, d'une sonde de température, d'une alimentation en éthanol (2) et en acide lactique (3) ;
- une première colonne de distillation (4) avec garnissage, rebouilleur, condenseur et système de reflux alimentée sous forme gazeuse via la conduite d'alimentation (5) en provenance du réacteur, munie d'une sortie en tête (6) du mélange éthanol - eau - lactate d'éthyle et d'une sortie en pied (7) du lactate d'éthyle contenant de l'acide lactique;
- une seconde colonne de distillation (8) avec garnissage surmontée également d'un rebouilleur, d'un condenseur et d'un système de reflux alimentée en phase liquide par les condensats de tête de la première colonne via la conduite d'alimentation (6), munie d'une sortie en tête (9) du mélange éthanol - eau et d'une sortie (10) contenant le lactate d'éthyle purifié.

Les exemples qui suivent illustrent la présente invention.

### Exemples

### Exemple 1

On introduit au préalable dans le réacteur d'estérification, d'une contenance de 9m³, une charge de 500 kg d'acide lactique de concentration 100% massique. Le produit est agité et chauffé à 100°C sous une pression réduite de 27,5 kPa absolu.

Quand l'acide lactique est à température, on introduit en continu 165,5 kg/h d'éthanol anhydre et 100 kg/h d'acide lactique 100%, équivalent à un ratio molaire éthanol/acide lactique de 3,6:1. L'estérification se déroule à 130°C, sous une pression réduite de 27,5 kPa absolu.

Une partie de l'éthanol réagit avec l'acide lactique pour produire du lactate d'éthyle et de l'eau, l'autre partie est utilisée comme agent strippant pour favoriser l'extraction des volatils du milieu réactionnel. Cela induit donc que la réaction d'estérification a lieu en excès d'acide lactique dans le réacteur.

On extrait en continu du milieu réactionnel la phase volatile comprenant l'eau, l'éthanol, le lactate d'éthyle et des traces d'acide lactique. La température de ces vapeurs s'élève à 120°C. Ce mélange présente une acidité inférieure ou égale à 0,2% (équivalent à 100% en lactate d'éthyle).

Cette phase gazeuse est injectée en continu dans une première colonne de distillation où elle subit une distillation sous pression réduite (27,5 kPa absolu). La colonne travaille sous un taux de reflux de 2. Une première fraction récoltée en tête, à une température de 70°C, est composée de :
- 47% d'éthanol,
- 21 % d'eau,
- 32% de lactate d'éthyle

La fraction en pied de colonne est récoltée à une température de 137°C et est constituée de 10% d'acide lactique et 90% de lactate d'éthyle. Ce mélange est recyclé vers le réacteur d'estérification.

La fraction récoltée en tête de la première colonne est ensuite injectée sous forme liquide dans une seconde colonne de distillation afin de purifier l'ester. Cette colonne travaille sous une pression de 10 kPa absolu et un taux de reflux de 0,2. L'ester purifié est récolté en pied de colonne à une température de 82°C et est composé de lactate d'éthyle de pureté supérieure à 97%. Le mélange éthanol - eau est récupéré en tête de colonne à une température de 30°C.

L'évolution de la composition du lactate d'éthyle récolté dans le rebouilleur en pied de seconde colonne est reprise dans le tableau suivant :

| Temps | Teneur en eau (%) | Acidité (%) | Couleur (Hazen) | Teneur en éthanol (%) | Teneur en lactate d'éthyle (%) |
|---|---|---|---|---|---|
| Phase démarrage et mise en régime des colonnes | | | | | |
| 5 heures | 0,12 | 0,04 | 5 | 0,2 | 99,64 |
| 8 heures | 0,08 | 0,02 | 13 | 0,1 | 99,88 |
| 10 heures | 0,06 | 0,05 | 9 | 0,5 | 99,39 |
| 24 heures | 0,1 | 0,06 | 5 | 0,3 | 99,54 |
| 2 jours | 0,08 | 0,01 | 12 | 0,1 | 99,81 |
| 3 jours (*) | 0,05 | 0,07 | 9 | 0,4 | 99,48 |
| 3 jours | 0,05 | 0,09 | 18 | 0,4 | 99,46 |
| 4 jours | 0,11 | 0,05 | 11 | 0,2 | 99,64 |

| | | | | | |
|---|---|---|---|---|---|
| (*) Échantillon prélevé directement en sortie de garnissage et non dans le rebouilleur. | | | | | |

### Exemple 2

Dans cet exemple, le procédé exposé à l'exemple 1 est renouvelé avec un éthanol contenant 70% d'eau.

La fraction récoltée en tête de la première colonne, à une température de 70°C est dans ce cas composée de :
- 14,1% d'éthanol,
- 69,9 % d'eau
- 16% de lactate d'éthyle

On peut donc voir que le procédé permet l'utilisation d'un éthanol chargé en eau mais que l'importance de sa teneur impacte la productivité en lactate d'éthyle.

### Exemple 3

Lors d'essais en ballons de distillation batch d'un mélange éthanol - eau - lactate d'éthyle - acide lactique, nous avons constaté à plusieurs reprises que la distillation de l'éthanol puis de l'eau s'effectuait correctement mais que, dès la disparition de ceux-ci, la distillation du lactate d'éthyle ne fonctionnait pas correctement. On observe que la pureté du lactate d'éthyle obtenu en tête diminue dans le temps et que le taux d'oligomère en pied de colonne augmente de manière significative.
Nous avons supposé que la présence d'acide lactique catalysait la réaction d'oligomérisation du lactate d'éthyle et que cette dernière était nettement amplifiée lorsque le milieu réactionnel se retrouvait dépourvu d'eau et d'éthanol, seuls composés capables d'hydrolyser ou de transestérifier les oligomères formés.

Afin de vérifier cet effet, nous avons effectué plusieurs expériences de distillation à pression atmosphérique sur des mélanges synthétiques.

Le dispositif expérimental est constitué d'un ballon de 500 ml destiné à recevoir le mélange à distiller. Ce dernier est inséré dans un chauffe-ballon agité dont la puissance de chauffe est réglée à son maximum. La colonne est remplie d'un garnissage non structuré et est isolée de l'air ambiant. En tête de colonne et dans le ballon sont disposé deux thermomètres permettant de suivre l'évolution de la température des vapeurs et celle du mélange. Les vapeurs sont récupérées dans un condenseur à eau. Les condensats sont récupérés dans un ballon de 250 ml et régulièrement pesés. La colonne utilisée ne comporte pas de reflux.

Les mélanges de synthèse contiennent tous initialement 10% d'éthanol, 30% d'eau et au minimum 58% de lactate d'éthyle en fonction de la quantité d'acide lactique introduite (2%, 1%, 0,5% et 0,2% en masse).

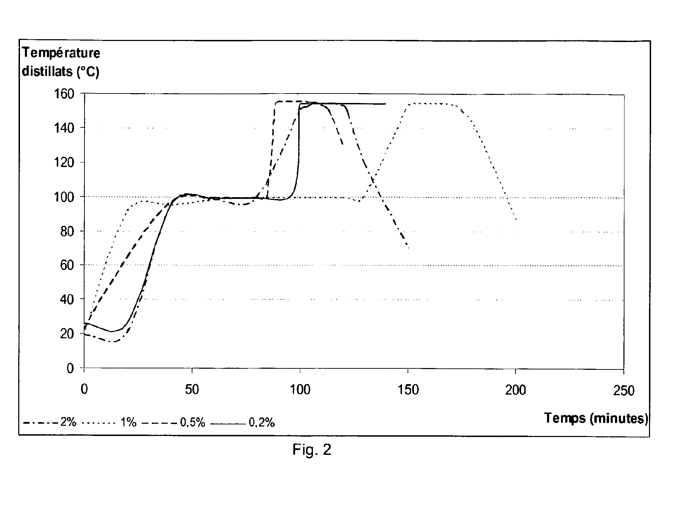

La figure 2 reprend l'évolution de la température des vapeurs en tête de colonne pour chaque test de distillation en fonction du temps.

Nous remarquons que, si la première partie de la séparation s'effectue correctement (extraction de l'éthanol et de l'eau), la distillation du lactate d'éthyle est plus délicate. Si nous suivons l'évolution de la température en tête de la colonne à distiller en fonction du temps, nous retrouvons trois paliers de température correspondant aux températures d'ébullition de l'éthanol pur et, très proche, de l'azéotrope eau/éthanol (78°C), de l'eau pure (100°C) et du lactate d'éthyle pur (154°C). Lors de ces tests, nous constatons que pendant 15 minutes, la température en tête reste constante, proche de 154°C puis chute assez rapidement.

Par contre, pour le mélange contenant initialement 0,2% d'acide lactique, cette température en tête reste constante. Il s'agit de la valeur seuil en dessous de laquelle la présence d'acide lactique ne perturbe plus la distillation.

De ces tests, nous avons ainsi déterminé la teneur maximale en acide lactique dans la fraction recueillie en tête de la première colonne de distillation, teneur idéalement inférieure ou égale à 0,2%.

### Exemple 4

Dans cet exemple, la procédure exposée à l'exemple 1 est renouvelée dans le cas du méthanol et d'un excès d'acide lactique.

Les résultats obtenus après 24h et 3jours de fonctionnement sont repris dans le tableau suivant :

| Temps | Teneur en eau (%) | Acidité (%) | Couleur (Hazen) | Teneur en lactate de méthyle (%) |
|---|---|---|---|---|
| 24 heures | 0,2 | 0,17 | 7 | 99,09 |
| 3 jours | 0,3 | 0,12 | 9 | 99,58 |

### Exemple 5

La procédure est similaire à l'exemple 1 pour la synthèse du lactate de butyle au départ de n-butanol et d'un excès d'acide lactique.

L'ester obtenu après 24h de fonctionnement présente une pureté de 99,5%, une acidité de 0,05% et une teneur en eau s'élevant à 0,25%

### Exemple 6

Le procédé exposé à l'exemple 1 est réitéré afin de synthétiser du lactate de 2-éthylhexanol.

Les résultats obtenus après plusieurs jours de fonctionnement sont repris dans le tableau suivant :

| Temps | Teneur en eau (%) | Acidité (%) | Couleur (Hazen) | Teneur en lactate de 2-éthylhexanol (%) |
|---|---|---|---|---|
| 1 jour | 0,02 | 0,03 | 13 | 99,95 |
| 6 jours | 0,11 | 0,07 | 20 | 99,82 |

## Revendications

1. Procédé continu d'obtention d'un ester lactique **caractérisé en ce qu'**il comporte les étapes suivantes :
a) Réaction d'estérification d'une composition comportant un alcool en présence d'une composition comportant de l'acide lactique en excès ;
b) Extraction d'une phase vapeur de ce milieu réactionnel, chargée en ester lactique, alcool, eau et des traces d'acide lactique ;
c) Distillation de la phase vapeur obtenue à l'étape b) pour récupérer en tête l'ester lactique, l'alcool et l'eau ;
d) Distillation de la fraction obtenue de l'étape c) pour récupérer en pied l'ester lactique purifié.

2. Procédé selon la revendication 1 **caractérisé en ce que** l'alcool contient de 1 à 12 carbones tel que le méthanol, l'éthanol, le n-butanol, l'isobutanol, le sec-butanol, le tert-butanol, le n-propanol, l'isopropanol, le 2-éthylhexanol, le 2-éthylbutanol, l'hexanol.

3. Procédé selon l'une quelconque des revendications précédentes **caractérisé en ce que** la composition d'acide lactique présente une concentration en acide comprise entre 50 et 100% massique dans de l'eau.

4. Procédé selon l'une quelconque des revendications précédentes **caractérisé en ce que** la composition d'alcool présente une concentration comprise en alcool entre 30% et 100% massique dans de l'eau.

5. Procédé selon l'une quelconque des revendications précédentes **caractérisé en ce que** la réaction d'estérification est réalisée sous pression de 15 à 300 kPa absolu.

6. Procédé selon l'une quelconque des revendications précédentes **caractérisé en ce que** l'estérification est réalisée à une température comprise entre 110 et 200°C.

7. Procédé selon l'une quelconque des revendications précédentes **caractérisé en ce que** le mélange réactionnel d'estérification à un rapport molaire acide lactique/alcool compris entre 1,1:1 à 50:1.

8. Procédé selon l'une quelconque des revendications précédentes **caractérisé en ce que** la réaction d'estérification est réalisée avec un rapport molaire du mélange alcool/acide lactique alimenté en continu compris entre 1 :1 et 5:1.

9. Procédé selon l'une quelconque des revendications précédentes **caractérisé en ce que** la fraction extraite en tête de la première colonne de distillation a une acidité inférieure à 0,5% massique (pour 100% d'ester lactique).

10. Procédé selon l'une quelconque des revendications précédentes **caractérisé en ce que** l'ester lactique purifié présente une pureté égale ou supérieure à 97%.

## Claims

1. Continuous method for obtaining a lactic ester, **characterised in that** it comprises the following steps:
a) esterification reaction of a composition comprising an alcohol in the presence of a composition comprising lactic acid in excess,
b) extraction of a vapour phase from this reaction medium, containing lactic ester, alcohol, water and traces of lactic acid;
c) distillation of the vapour phase obtained at step b) in order to recover the lactic ester, alcohol and water at the top;
d) distillation of the fraction obtained at step c) in order to recover the purified lactic ester at the bottom.

2. Method according to claim 1, **characterised in that** the alcohol contains 1 to 12 carbons such as methanol, ethanol, n-butanol, isobutanol, sec-butanol, tert-butanol, n-propanol, isopropanol, 2-ethylhexanol, 2-ethylbutanol or hexanol.

3. Method according any one of the preceding claims, **characterised in that** the lactic acid composition has a concentration of acid of between 50% and 100% by weight in water.

4. Method according to any one of the preceding claims, **characterised in that** the alcohol composition has an alcohol composition of between 30% and 100% by weight in water.

5. Method according to any one of the preceding claims, **characterised in that** the esterification reaction is carried out at a pressure of 15 to 300 kPa absolute.

6. Method according to any one of the preceding claims, **characterised in that** the esterification is performed at a temperature between 110° and 200°C.

7. Method according to any one of the preceding claims, **characterised in that** the esterification reaction mixture has a lactic acid/alcohol mol ratio of between 1.1:1 and 50:1.

8. Method according to any one of the preceding claims, **characterised in that** the esterification reaction is performed with a mol ratio of the alcohol/lactic acid mixture supplied continuously between 1:1 and 5:1.

9. Method according to any one of the preceding claims, **characterised in that** the fraction extracted at the head of the first distillation column has an acidity of less than 0.5% by weight (for 100% lactic ester).

10. Method according to any one of the preceding claims, **characterised in that** the purified lactic ester has a purity equal to or greater than 97%.

## Patentansprüche

1. Kontinuierliches Verfahren zur Gewinnung eines Milchsäureesters, **dadurch gekennzeichnet, dass** es die folgenden Schritte umfasst:
a) Veresterungsreaktion einer Zusammensetzung, umfassend ein Alkohol in Gegenwart einer Zusammensetzung mit überschüssiger Milchsäure;
b) Extraktion einer Dampfphase aus diesem Reaktionsmedium, beladen mit Milchsäureester, Alkohol, Wasser und Spuren von Milchsäure;
c) Destillation der Dampfphase, erhalten in Schritt b), um den Milchsäureester, den Alkohol und das Wasser am Kopf wiederzugewinnen;
d) Destillation der Fraktion, erhalten in Schritt c), um am Fuß den gereinigten Milchsäureester wiederzugewinnen.

2. Verfahren nach Anspruch 1, **dadurch gekennzeichnet, dass** der Alkohol 1 bis 12 Kohlenstoffe enthält wie z.B. Methanol, Ethanol, n-Butanol, Isobutanol, sec-Butanol, tert-Butanol, n-Propanol, Isopropanol, 2-Ethylhexanol, 2-Ethylbuntanol, Hexanol.

3. Verfahren nach einem der vorhergehenden Ansprüche, **dadurch gekennzeichnet, dass** die Zusammensetzung aus Milchsäure eine Säurekonzentration zwischen 50 und 100 Massenanteilen in Wasser aufweist.

4. Verfahren nach einem der vorhergehenden Ansprüche, **dadurch gekennzeichnet, dass** die Zusammensetzung aus Alkohol eine Alkoholkonzentration zwischen 30 und 100 Massenanteilen in Wasser aufweist.

5. Verfahren nach einem der vorhergehenden Ansprüche, **dadurch gekennzeichnet, dass** die Veresterungsreaktion unter einem absoluten Druck von 15 bis 300 kPa erfolgt.

6. Verfahren nach einem der vorhergehenden Ansprüche, **dadurch gekennzeichnet, dass** die Veresterung bei einer Temperatur zwischen 110 und 200 °C erfolgt.

7. Verfahren nach einem der vorhergehenden Ansprüche, **dadurch gekennzeichnet, dass** die Veresterungsreaktionsmischung ein Molverhältnis Milchsäure/Alkohol zwischen 1,1:1 und 50:1 aufweist.

8. Verfahren nach einem der vorhergehenden Ansprüche, **dadurch gekennzeichnet, dass** die Veresterungsreaktion mit einem Molverhältnis der Mischung Alkohol/Milchsäure, kontinuierlich beschickt, zwischen 1:1 und 5:1 erfolgt.

9. Verfahren nach einem der vorhergehenden Ansprüche, **dadurch gekennzeichnet, dass** die am Kopf der ersten Destilationssäule extrahierte Fraktion einen Säuregehalt von weniger als 0,5 Massenanteile (für 100 % Milchsäureester) aufweist.

10. Verfahren nach einem der vorhergehenden Ansprüche, **dadurch gekennzeichnet, dass** der gereinigte Milchsäureester eine Reinheit gleich oder höher als 97 % aufweist.
